(19) Europäisches Patentamt / European Patent Office / Office européen des brevets

(11) EP 2 045 264 B1

(12) **EUROPEAN PATENT SPECIFICATION**

(45) Date of publication and mention of the grant of the patent:
**26.10.2011 Bulletin 2011/43**

(51) Int Cl.:
***C07K 14/435*** *(2006.01)* ***G01N 33/52*** *(2006.01)*
***G01N 33/84*** *(2006.01)*

(21) Application number: **08165522.7**

(22) Date of filing: **30.09.2008**

(54) **Novel pH- and anion concentration-responsive GFP mutant, a chimeric protein comprising such a mutant and a method for the combined assaying of the pH and anion concentration**

Neue, auf pH- und Anionenkonzentration reagierende GFP-Mutante, chimäres Protein mit solch einer Mutante und Verfahren zur kombinierten Analyse der pH- und Anionenkonzentration

Nouveau mutant GFP sensible au pH et à la concentration en anions, et protéine chimérique comprenant ledit mutant et procédé d'analyse combinée du pH et de la concentration en anions

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **01.10.2007 IT TO20070687**

(43) Date of publication of application:
**08.04.2009 Bulletin 2009/15**

(73) Proprietor: **Consiglio Nazionale Delle Ricerche**
**00185 Roma (IT)**

(72) Inventors:
- **Arosio, Daniele**
  **38123 Trento (IT)**
- **Beltram, Fabio**
  **34170 Gorizia (IT)**
- **Ricci, Fernanda**
  **55100 Lucca- Nave (IT)**
- **Marchetti, Laura**
  **35010 Borgoricco (Padova) (IT)**

(74) Representative: **Comoglio, Elena et al**
**Jacobacci & Partners S.p.A.**
**Corso Emilia 8**
**10152 Torino (IT)**

(56) References cited:
**EP-A- 1 178 109** **EP-A- 1 264 888**
**WO-A-03/025220** **WO-A-2005/024003**

- **CHUDAKOV ET AL: "Fluorescent proteins as a toolkit for in vivo imaging" TRENDS IN BIOTECHNOLOGY, ELSEVIER PUBLICATIONS, CAMBRIDGE, GB, vol. 23, no. 12, 1 December 2005 (2005-12-01), pages 605-613, XP005182978 ISSN: 0167-7799**
- **AROSIO DANIELE ET AL: "Spectroscopic and structural study of proton and halide ion cooperative binding to gfp." BIOPHYSICAL JOURNAL 1 JUL 2007, vol. 93, no. 1, 1 July 2007 (2007-07-01), pages 232-244, XP002510118 ISSN: 0006-3495**
- **BIZZARRI RANIERI ET AL: "Green fluorescent protein ground states: The influence of a second protonation site near the chromophore" BIOCHEMISTRY, vol. 46, no. 18, May 2007 (2007-05), pages 5494-5504, XP002510119 ISSN: 0006-2960**
- **VINKENBORG JAN L ET AL: "Enhanced sensitivity of FRET-based protease sensors by redesign of the GFP dimerization interface" CHEMBIOCHEM, vol. 8, no. 10, July 2007 (2007-07), pages 1119-1121, XP002510120 ISSN: 1439-4227**
- **AROSIO DANIELE P: "Heterotropic Linkage between Halide and Proton ion binding to a GFP-based halide indicator for in vivo study." BIOPHYSICAL JOURNAL, no. Suppl. S, January 2007 (2007-01), page 386A, XP002518891 & 51ST ANNUAL MEETING OF THE BIOPHYSICAL-SOCIETY; BALTIMORE, MD, USA; MARCH 03 -07, 2007 ISSN: 0006-3495**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- BIZZARRI RANIERI ET AL: "Development of a novel GFP-based ratiometric excitation and emission pH indicator for intracellular studies" BIOPHYSICAL JOURNAL, vol. 90, no. 9, May 2006 (2006-05), pages 3300-3314, XP002518892 ISSN: 0006-3495
- AROSIO DANIELE ET AL: "Fluorescence Biosensor Yields pH and Chloride Concentration for In Vivo Systems" BIOPHYSICAL JOURNAL; JOINT 52ND ANNUAL MEETING OF THE BIOPHYSICAL SOCIETY AND 16TH INTERNATIONAL BIOPHYSICS CONGRESS, NEW YORK, US, US; LONG BEACH CONVENTION CENTER,LONG BEACH, CALIFORNIA (USA), vol. 94, no. Suppl, 5 February 2008 (2008-02-05), page 2240, XP008099174 ISSN: 0006-3495
- MARKOVA ET AL: "Genetically encoded chloride indicator with improved sensitivity" JOURNAL OF NEUROSCIENCE METHODS, ELSEVIER SCIENCE PUBLISHER B.V., AMSTERDAM, NL, vol. 170, no. 1, 6 January 2008 (2008-01-06), pages 67-76, XP022559936 ISSN: 0165-0270

SEQUENCE LISTING

<110> Consiglio Nazionale delle Ricerche - CNR istituto nazionale per la fisica della materia - INFM

<120> A novel GFP-mutant sensitive to pH and anion concentration, a chimeric protein comprising such a mutant and a method for the combined detection of pH and anion concentration

<130> E070957-EC

<150> TO2007A000687
<151> 2007-10-01

<160> 6

<170> PatentIn version 3.3

<210> 1
<211> 238
<212> PRT
<213> Aequorea victoria

<400> 1

Met Ser Lys Gly Glu Glu Leu Phe Thr Gly Val Val Pro Ile Leu Val
1               5                   10                  15

Glu Leu Asp Gly Asp Val Asn Gly His Lys Phe Ser Val Ser Gly Glu
            20                  25                  30

Gly Glu Gly Asp Ala Thr Tyr Gly Lys Leu Thr Leu Lys Phe Ile Cys
        35                  40                  45

Thr Thr Gly Lys Leu Pro Val Pro Trp Pro Thr Leu Val Thr Thr Phe
    50                  55                  60

Ser Tyr Gly Val Gln Cys Phe Ser Arg Tyr Pro Asp His Met Lys Gln
65                  70                  75                  80

His Asp Phe Phe Lys Ser Ala Met Pro Glu Gly Tyr Val Gln Glu Arg
                85                  90                  95

Thr Ile Phe Phe Lys Asp Asp Gly Asn Tyr Lys Thr Arg Ala Glu Val
            100                 105                 110

Lys Phe Glu Gly Asp Thr Leu Val Asn Arg Ile Glu Leu Lys Gly Ile
        115                 120                 125

Asp Phe Lys Glu Asp Gly Asn Ile Leu Gly His Lys Leu Glu Tyr Asn
    130                 135                 140

Tyr Asn Ser His Asn Val Tyr Ile Met Ala Asp Lys Gln Lys Asn Gly
145                 150                 155                 160

Ile Lys Val Asn Phe Lys Ile Arg His Asn Ile Glu Asp Gly Ser Val
                165                 170                 175

Gln Leu Ala Asp His Tyr Gln Gln Asn Thr Pro Ile Gly Asp Gly Pro
            180                 185                 190

Val Leu Leu Pro Asp Asn His Tyr Leu Ser Thr Gln Ser Ala Leu Ser
        195                 200                 205

Lys Asp Pro Asn Glu Lys Arg Asp His Met Val Leu Leu Glu Phe Val
    210                 215                 220

Thr Ala Ala Gly Ile Thr His Gly Met Asp Glu Leu Tyr Lys
225                 230                 235

<210> 2
<211> 238
<212> PRT
<213> Aequorea victoria

<400> 2

Met Ser Lys Gly Glu Glu Leu Phe Thr Gly Val Val Pro Ile Leu Val
1               5                   10                  15

Glu Leu Asp Gly Asp Val Asn Gly His Lys Phe Ser Val Ser Gly Glu
            20                  25                  30

Gly Glu Gly Asp Ala Thr Tyr Gly Lys Leu Thr Leu Lys Phe Ile Cys
        35                  40                  45

Thr Thr Gly Lys Leu Pro Val Pro Trp Pro Thr Leu Val Thr Thr Leu
    50                  55                  60

Thr Tyr Gly Val Gln Cys Phe Ser Arg Tyr Pro Asp His Met Lys Gln
65                  70                  75                  80

His Asp Phe Phe Lys Ser Ala Met Pro Glu Gly Tyr Val Gln Glu Arg
                85                  90                  95

Thr Ile Phe Phe Lys Asp Asp Gly Asn Tyr Lys Thr Arg Ala Glu Val
            100                 105                 110

Lys Phe Glu Gly Asp Thr Leu Val Asn Arg Ile Glu Leu Lys Gly Ile
        115                 120                 125

Asp Phe Lys Glu Asp Gly Asn Ile Leu Gly His Lys Leu Glu Tyr Asn
    130                 135                 140

Tyr Asn Ser His Asn Val Tyr Ile Met Ala Asp Lys Gln Lys Asn Gly
145                 150                 155                 160

Ile Lys Val Asn Phe Lys Ile Arg His Asn Ile Glu Asp Gly Ser Val
                165                 170                 175

Gln Leu Ala Asp His Tyr Gln Gln Asn Thr Pro Ile Gly Asp Gly Pro
            180                 185                 190

Val Leu Leu Pro Asp Asn His Tyr Leu Ser Tyr Gln Ser Ala Leu Ser
        195                 200                 205

Lys Asp Pro Asn Glu Lys Arg Asp His Met Val Leu Leu Glu Phe Val
    210                 215                 220

Thr Ala Ala Gly Ile Thr Leu Gly Met Asp Glu Leu Tyr Lys
225                 230                 235

<210> 3
<211> 238
<212> PRT
<213> Aequorea victoria

<400> 3

```
Met Ser Lys Gly Glu Glu Leu Phe Thr Gly Val Val Pro Ile Leu Val
1               5                   10                  15

Glu Leu Asp Gly Asp Val Asn Gly His Lys Phe Ser Val Ser Gly Glu
            20                  25                  30

Gly Glu Gly Asp Ala Thr Tyr Gly Lys Leu Thr Leu Lys Phe Ile Cys
        35                  40                  45

Thr Thr Gly Lys Leu Pro Val Pro Trp Pro Thr Leu Val Thr Thr Leu
    50                  55                  60

Thr Tyr Gly Val Gln Cys Phe Ser Arg Tyr Pro Asp His Met Lys Gln
65                  70                  75                  80

His Asp Phe Phe Lys Ser Ala Met Pro Glu Gly Tyr Val Gln Glu Arg
                85                  90                  95

Thr Ile Phe Phe Lys Asp Asp Gly Asn Tyr Lys Thr Arg Ala Glu Val
            100                 105                 110

Lys Phe Glu Gly Asp Thr Leu Val Asn Arg Ile Glu Leu Lys Gly Ile
        115                 120                 125

Asp Phe Lys Glu Asp Gly Asn Ile Leu Gly His Lys Leu Glu Tyr Asn
    130                 135                 140

Tyr Asn Ser His Asn Val Tyr Ile Met Ala Asp Lys Gln Lys Asn Gly
145                 150                 155                 160

Ile Lys Val Asn Phe Lys Ile Arg His Asn Ile Glu Asp Gly Ser Val
                165                 170                 175

Gln Leu Ala Asp His Tyr Gln Gln Asn Thr Pro Ile Gly Asp Gly Pro
            180                 185                 190

Val Leu Leu Pro Asp Asn His Tyr Leu Ser Tyr Gln Ser Ala Leu Ser
        195                 200                 205

Lys Asp Pro Asn Glu Lys Arg Asp His Met Val Leu Leu Glu Phe Leu
    210                 215                 220

Thr Ala Ala Gly Ile Thr Leu Gly Met Asp Glu Leu Tyr Lys
225                 230                 235
```

<210> 4
<211> 753
<212> DNA
<213> Aequorea victoria

<400> 4

```
atggctagct ggagccaccc gcagttcgaa aaaggcgccg tgagcaaggg cgaggagctg      60
ttcaccgggg tggtgcccat cctggtcgag ctggacggcg acgtaaacgg ccacaagttc     120
agcgtgtccg gcgagggcga gggcgatgcc acctacggca agctgaccct gaagttcatc     180
tgcaccaccg gcaagctgcc cgtgccctgg cccaccctcg tgaccaccct gacctacggc     240
gtgcagtgct tcagccgcta ccccgaccac atgaagcagc acgacttctt caagtccgcc     300
atgcccgaag gctacgtcca ggagcgcacc atcttcttca aggacgacgg caactacaag     360
acccgcgccg aggtgaagtt cgagggcgac accctggtga accgcatcga gctgaagggc     420
atcgacttca aggaggacgg caacatcctg gggcacaagc tggagtacaa ctacaacagc     480
cacaacgtct atatcatggc cgacaagcag aagaacggca tcaaggtgaa cttcaagatc     540
cgccacaaca tcgaggacgg cagcgtgcag ctcgccgacc actaccagca gaacaccccc     600
atcggcgacg gccccgtgct gctgcccgac aaccactacc tgagctacca gtccgccctg     660
agcaaagacc ccaacgagaa gcgcgatcac atggtcctgc tggagttcct gaccgccgcc     720
gggatcactc tcggcatgga cgagctgtac aag                                  753
```

<210> 5
<211> 20
<212> PRT
<213> Artificial

<220>
<223> Linker peptide

<400> 5

```
Arg Gly Ser Ala Ser Gly Gly Gly Gly Gly Leu Val Pro Arg Gly Ser
1               5                   10                  15

Ala Ser Gly Ala
            20
```

<210> 6
<211> 60
<212> DNA
<213> Artificial

<220>
<223> Linker peptide nucleic acid sequence

<400> 6

```
cgcggatccg cgtctggtgg tggtggtggt ctagttccac gtggatctgc ctcaggagca      60
```

## Description

The present invention generally refers to a novel pH- and anion concentration-responsive fluorescent protein, a chimeric protein comprising such a fluorescent protein and a method for the combined assaying of the pH and anion concentration by using such a chimeric protein.

More particularly, the novel fluorescent protein of the invention is a GFP ("Green Fluorescent Protein") protein mutant.

The native form of the GFP protein ("wild-type-GFP, GFP) is a jellyfish protein (*Aequorea victoria*) of 238 amino acids in length and 27 kD in molecular weight, responsible for the green luminescence of the animal through a mechanism of photo-conversion of the blue light emitted by a second calcium-activated protein called aequorin. The characterisation of the GFP protein has demonstrated that its optical properties are independent from cofactors and its fluorescence can be excited independently from aequorin with a characteristic wavelength radiation.

The amino acid sequence of the native GFP (wtGFP) is here set forth in the section entitled "Sequence Listing" as SEQ ID NO:1.

Several mutants of the wild-type GFP protein are described in the prior art, including the one designated as E2GFP, with the amino acid sequence set forth in the Sequence Listing as SEQ ID NO:2. Such a mutant is described in the Italian patent IT 1320791, which designates some of the present inventors, wherein also the nucleotide sequence encoding for the E2GFP protein is provided.

The present inventors continued the studies concerning the GFP mutants, the E2GFP protein in particular, and have verified that the S65T/T203Y double mutation contained therein causes a marked sensitivity of its fluorescence to the pH and concentration of anions. In this respect, see for instance Arosio D, Garau G, Ricci F, Marchetti L, Bizzarri R, Nifosi R, Beltram F. Biophys J. 2007 Jul 1;93(1):232-44. Epub 2007 Apr 13.

Sensors based on fluorescent proteins responsive to chemical parameters such as pH and anion concentration are described in the prior art. The European patent application EP 1514922 describes a method of monitoring the changes in the concentration of anions or the changes in pH, by using a construct comprising a first fluorescent protein that is a mutant of the Enhanced Yellow Fluorescent Protein (EYFP) as the sensor element, the said mutant being designed so as to bind the anions and having a fluorescence that is sensitive to the pH, and a second fluorescent protein designed to produce FRET with the EYFP mutant, the first and second protein being connected through a peptide linker.

The sensor system described in EP 1514922 allows the changes in pH or, alternatively, the changes in the concentration of anions to be monitored, but does not allow to do combined measurements of the pH and anion concentration.

In the above-mentioned article to Arosio D., et al., 2007, the authors hint at the possibility of using the E2GFP protein as a combined sensor for pH and chlorine ions. However, this article does not indicate how such a combined sensor can be obtained.

The prior art relating to this field is thus characterised by severe limitations, which are overcome by the novel GFP protein mutant, object of the present invention, and by the chimeric protein comprising the said mutant.

Thus, a first object of the invention is a mutant of the wild-type GFP protein with the amino acid sequence depicted in the Sequence Listing as SEQ ID NO: 3, designated as D3GFP.

The D3GFP sequence derives from the wild-type GFP sequence as a result of the following substitutions:

(i) substitution of the phenylalanine residue (F) at position 64 with a leucine residue (L) [F64L];
(ii) substitution of the serine residue (S) at position 65 with a threonine residue (T) [S65T];
(iii) substitution of the threonine residue (T) at position 203 with a tyrosine residue (Y) [T203Y]; and
(iv) substitution of the valine residue (V) at position 224 with a leucine residue (L) [V224L].

The main features of the D3GFP mutant of the invention are the ability to bind chlorine ions with a very high affinity and the sensitivity of the fluorescence to both the pH and anion concentration. The affinity of D3GFP for chlorine ions is extremely high, although it changes depending on the pH. The dissociation constant of the binding of chlorine ions to D3GFP at pH < 6.0 as calculated by the present inventors is approximately 1 mM, that is a one order of magnitude lower than the corresponding value calculated for the known E2GFP mutant (${}^{Cl}K_d \approx$ 12-15 mM) (cf. figure 1). This means that the affinity of the D3GFP mutant of the invention for chlorine ions exceeds that of the known E2GFP mutant by approximately one order of magnitude. As will be explained in detail below, these features make the D3GFP mutant particularly suited to be used in the construction of a sensor designed to measure both the pH and anion concentration, chlorine ions in particular, in a sample, with a high sensitivity.

The D3GFP mutant of the present invention can be obtained by mutagenesis and recombinant DNA techniques *per se* known to the person skilled in the art.

The mutagenesis techniques contemplate the generation of mutations in a known starting nucleotide sequence - for instance the sequence encoding for the GFP protein or a mutant thereof such as E2GFP or EGFP - which cause the modification of the amino acid sequence of the protein encoded by the nucleotide sequence. The mutagenesis techniques comprise, for example, the site-directed mutagenesis.

By using the mutagenesis techniques, a mutated nucleic acid is obtained which has a nucleotide sequence encoding for the amino acid sequence of the D3GFP mutant as described above.

Example 1 illustrates the preparation of the nucleotide sequence encoding for the D3GFP mutant of the invention starting from the gene encoding for the EGFP protein - the amino acid sequence of which differs from the wild-type GFP one in the two substitutions F64L and S65T - and the successive amplification by PCR. The example is given solely by way of illustration and is not intended to be limiting in any way, in that the selection of the mutagenesis method used is not critical in achieving the desired result.

Thus, a second object of the invention is an isolated nucleic acid comprising a sequence encoding for the D3GFP mutant of the invention. Such a sequence encoding for D3GFP preferably has the nucleotide sequence depicted in SEQ LID NO: 4.

The D3GFP protein of the invention is generated, for example, by the recombinant DNA technology starting from the nucleic acid obtained through mutagenesis. To this end, the nucleotide sequence encoding for D3GFP is inserted into an expression vector, that is a molecular structure containing at least the sequence of a constitutive or inducible promoter capable of facilitating the transcription of the inserted gene. The expression vector is in turn inserted into a target cell, also known as host cell, in which it is able to induce the expression of the encoded peptide sequence. As known, expression vectors may also include expression control sequences operably linked to the sequence encoding for the protein product of interest. Therefore, an expression vector comprising a nucleotide sequence encoding for the D3GFP protein as defined above and a transformed host cell comprising such an expression vector fall within the scope of the present invention.

Different types of expression vectors are known, each of them adapted so as to work in the desired host cell type. Examples of vectors suitable for the transfer and expression of the nucleotide sequence of the invention are plasmid vectors, for instance constructed for the expression in bacterial or yeast cells, and virus vectors, for instance constructed for the expression in insect or mammalian cells. Examples of host cells suitable for the expression of the nucleotide sequence of the invention are prokaryotic cells, for instance bacterial cells such as *Escherichia coli,* and eukaryotic cells, for instance yeast cells such as *Saccharomyces cerevisiae* and *Pichia pastoris,* and insect cells, particularly cells infectable by recombinant baculoviruses. Such expression systems vector/host cell are also widely known in the prior art. The selection of a particular vector/host system is non critical in achieving the desired result, and is within the skills of the person of ordinary skill in the art. The transfer of the expression vector into the above-mentioned organisms can be carried out by any one of the known gene transfer techniques, for example the transfection of plasmid DNA by chemical, physical or viral methods.

Through the recombinant DNA technology it is therefore possible to obtain eukaryotic or prokaryotic cells transiently or stably transformed with a vector designed to express the D3GFP protein of interest. The D3GFP protein thus produced may be purified from the above-mentioned host cells. Examples of suitable purification methods are the purification by chromatographic techniques from E. *coli,* the fusion of the protein of interest with epitopes recognisable by monoclonal or polyclonal antibodies, the fusion of the protein of interest with protein domains that are able to bind to affinity columns (for example, poly-histidine sequences, glutathione-S-transferase, biotin mimetic peptides such as *strep-tag,* maltose binding protein, and the like).

As mentioned above, the particular fluorescence and chlorine ion binding characteristics of the D3GFP mutant of the present invention make this protein particularly suited to be used in the construction of a combined sensor for pH and anions.

To this end, a chimeric protein is constructed, which comprises a first fluorescent protein, designated as P1, and a second fluorescent protein, designated as P2.

P1 is a protein, the fluorescence characteristics of which change depending on both the pH and anion concentration. P1 is the D3GFP mutant of the invention.

P2 is a fluorescent protein, the fluorescence characteristics of which are independent from both the pH and anion concentration. Furthermore, P2 is selected so as there is no substantial overlapping of the P1 and P2 emission and excitation fluorescence spectra. This requirement is considered fulfilled when the energy transfer phenomenon conventionally designated as FRET does non occur between the two proteins P1 and P2 or, at most, when the FRET is so mild as to not significantly interfere with the fluorescence characteristics of the two proteins.

One example of the chimeric protein of the invention designed to be used as a combined sensor for pH and anions, chlorine ions in particular, is the chimeric protein consisting of the D3GFP mutant connected to the monomeric red fluorescent protein (DsRed-monomer, commercially available for instance from Clontech) through a short peptide sequence designated as "peptide linker". One example of the peptide linker suitable to this end is the 20 amino acid peptide depicted in SEQ ID NO:5.

Other proteins that can be used as the P2 protein in the chimeric protein of the invention are all the mRFP1-derived monomers, such as mTangerine, mStrawberry, mCherry, mGrapel, mRaspberry, mPlum, and also TdTomato, dTomato, [Shaner, N. C.; Campbell, R. E.; Steinbach, P. A.; Giepmans, B. N. G.; Palmer, A. E. & Tsien, R. Y. Improved monomeric red, orange and yellow fluorescent proteins derived from Discosoma sp. red fluorescent protein. Nat Biotechnol, 2004, 22, 1567-1572] DsRed [clontech] (Tsien Lab), or HcRed [evrogen] [Gurskaya, N. G.; Fradkov, A. F.; Terskikh, A.; Matz, M. V.; Labas, Y. A.; Martynov, V. I.; Yanushevich, Y. G.; Lukyanov, K. A. & Lukyanov, S. A. GFP-like chromoproteins as a source of far-red fluorescent proteins. FEBS Lett, Shemiakin and Ovchinnikov Institute of Bioorganic Chemistry RAS, Moscow, Russia., 2001, 507, 16-20], Red EosFP [Wiedenmann, J.; Ivanchenko, S.; Oswald, F.; Schmitt, F.; Röcker, C.; Salih, A.; Spindler, K. & Nienhaus, G. U. EosFP, a fluorescent marker protein with UV-inducible green-to-red fluorescence conversion. Proc Natl Acad Sci U S A, Department of General Zoology and Endocrinology, University of Ulm, 89069 Ulm, Germany., 2004, 101, 15905-15910].

The chimeric protein can be obtained by using methods known to the person skilled in the art, by constructing expression vectors as described above, the said vectors, besides the nucleic acid sequence encoding for the D3GFP mutant, also containing the nucleic acid sequence encoding for the P2 protein and the oligonucleotide encoding for the peptide linker. The nucleic acid sequences encoding for the P2 protein and the peptide linker can be placed under the control of the same regulatory sequences (for example, a promoter) that drive the expression of D3GFP. In this way it is possible to obtain expression vectors and prokaryotic or eukaryotic cells capable of stably or transiently expressing protein chimeras consisting of a fusion polypeptide that, besides the fluorescence characteristics of the D3GFP mutant, also exhibits the typical fluorescence characteristics of the P2 fluorescent protein.

Therefore, also an expression vector comprising a nucleic acid encoding for the chimeric protein as described above and a transformed host cell comprising such an expression vector fall within the scope of the invention.

The chimeric protein of the invention is particularly suited to be used as a sensor element for the combined measurement of the pH and anion concentration in a sample, particularly in a biological sample, such as for example a cell culture. Moreover, as the chimeric protein can be transfected into an eukaryotic or prokaryotic cell, either targeted to specific sub-cellular compartments through the fusion with specific targeting sequences or macromolecules, or distributed throughout the cell itself, the chimeric protein can be used advantageously for intracellular combined measurements of the pH and anion concentration, particularly chlorine ions. The use of the chimeric protein as a sensor is made possible by its particular fluorescence characteristics. Example 4 describes in detail the study of the fluorescence characteristics of the chimeric D3GFP-linker-DsRed-monomer protein (linker = SEQ ID NO:5), although the results obtained from such a study and the conclusions that can be derived therefrom have a general validity, in the sense that they can be applied to all of the chimeric proteins falling within the scope of the present invention.

In summary, the D3GFP-linker-DsRed-monomer excitation spectrum is characterised by a region where the fluorescence varies according to the pH and anion concentration and a region where the fluorescence is independent from the pH and anion concentration.

The analysis of the pH-dependence of the excitation spectrum (fig. 2B) shows the presence of an isosbestic point at around 470-475 nm with a pK of approximately 6.3, which makes the assaying of the pH possible in the range from about 4.5 to 8.5 at 37°C with excitation ratiometric measures. In particular, such an assaying can be carried out based on the value of a parameter of the excitation spectrum designated as $R_1$, which has the property of varying according to the pH but not according to the concentration of chlorine ions. By comparing the $R_1$ value determined for the test sample with a calibration curve previously generated by using reference samples of known pH, it is possible to determine the pH value of the test sample.

Preferably, $R_1$ is given by the following general formula:

$$R_1 = \frac{A}{B}$$

wherein A is the fluorescence emitted as a result of a 488 nm wavelength excitation and B is the fluorescence emitted as a result of a 458 nm wavelength excitation.

Preferably, the fluorescence obtained with the 488 nm wavelength excitation and the fluorescence obtained with the 458 nm wavelength excitation are measured within the 500-550 nm range emission.

Furthermore, the analysis of the chlorine ion concentration-dependence of the excitation spectrum (fig. 3) shows the existence of a correlation between the excitation fluorescence and the chlorine ion concentration, which makes the assaying of the chlorine ion concentration possible in the range from about 0.5 to 500 mM at 37°C with excitation ratiometric measures. In particular, such an assaying is carried out based on the value of a parameter of the excitation spectrum designated as $R_2$, which has the property of varying according to both the pH and the concentration of chlorine ions. By comparing the $R_2$ value determined for the test sample with a calibration curve previously generated by using reference samples of known chlorine ion concentrations, and the pH thereof having been determined based on the $R_1$

parameter, it is possible to determine the anion concentration in the test sample. Preferably, $R_2$ is given by the following general formula:

$$R_2 = \frac{(A + B)}{C}$$

wherein A and B are as defined above and C is the fluorescence emitted as a result of a 543 nm wavelength excitation.

As can be seen in fig. 3, the excitation fluorescence at the wavelength of 543 nm remains substantially unvaried when the concentration of chlorine varies. Thus, the fluorescence at $\lambda_{ex}$ = 543 nm is used for the normalisation.

The chimeric protein of the invention may be used for the combined assaying of the pH and anion concentration in any sample, preferably a biologically-derived sample. Furthermore, as such a protein can be transfected into a cell, such a protein can be used advantageously for intracellular combined measurements of the pH and anion concentration, particularly chlorine ions.

The following examples are given exclusively by way of illustration and are not to be construed as limiting the scope of the present invention in any way.

Example 1: Construction of the D3GFP mutant

The synthesis of the D3GFP mutant was carried out starting from the gene encoding for the E2GFP (ref. D. Arosio et al.) that contains the four amino acid substitutions F64L, S65T, T203Y and H231 L. The site-directed mutagenesis was achieved by the QuikChange® Site-Directed Mutagenesis kit (Stratagene), by using oligonucleotides designed to contain nucleotide substitutions capable of causing the amino acid substitution V224L (Forward: 5'-GTCCTGCTGGAGTTCCTGACCGCCGCCGGGATC-3' (SEQ ID NO:9)); Reverse: 5'- GATCCCGGCGGCGGTCAGGAACTCCAGCAGGAC-3' (SEQ ID NO:10)).

In this way, the nucleic acid encoding for the D3GFP protein with the sequence SEQ ID NO: 4 was obtained.

The encoding nucleic acid was amplified by PCR using oligonucleotides that introduce restriction sites compatible with those of an expression vector through standard molecular biology procedures. An 8 amino acid tail recognised by streptavidin (pPR-IBA 2, IBA GmbH, Germany) or a poly-histidine tail (pET-151/D-TOPO, Invitrogen) is encoded in the vector, so as to facilitate the purification of the expressed recombinant protein by affinity chromatography.

The expression vector containing the insert encoding for D3GFP was then transferred into competent E. *coli* by a classical heat-shock procedure. Following the reaction, the vector is recognised by the replicative and translational machinery of the bacterial cell. The vector contains an inducible promoter under the control of the inducer isopropyl-beta-D-thiogalactopyranoside (IPTG).

The highest protein expression from E. *coli* was obtained at 30°C, 20 hours post-induction.

The protein was extracted from the bacteria by a standard sonication procedure.

The purification of the protein from the bacterial lysate was obtained by a first chromatography through a streptavidin-enriched column (IBA GmbH) or through a nickel column (HiTrap, Pharmacia) and by a second purification through an ion-exchange column (Resource Q, Pharmacia), which separates the proteins according to their charge, reproducibly obtaining perfectly monodisperse samples with a high purity and concentration in an elution buffer (20 mM diethanolamine pH 8.6, 250 mM $Na_2SO_4$) lacking halogen ions, as required for the subsequent photo-physical and affinity studies on the halogens themselves.

The monomeric condition of the proteins was verified by size-exclusion chromatography.

Example 2: Cloning of the E2GFP-linker-DsRed-monomer construct

The tandem construct encoding for the chimeric E2GFP-linker-DsRed-monomer protein was obtained by a standard ligation reaction (catalyzed by the T4 DNA ligase enzyme) of the PCR product encoding for E2GFP (SEQ ID NO:2), the double-stranded oligonucleotide encoding for the peptide linker (SEQ ID NO:5) and the PCR product encoding for DsRed-monomer (SEQ ID NO:7). All the constructs were then sequenced throughout the entire length of the inserts (SEQ ID NO:8).

The construct thus obtained was amplified by PCR using oligonucleotides that introduce restriction sites compatible with those of the bacterial expression plasmid pBR-IBA 2 (IBA, GmbH) through standard molecular biology procedures.

The recombinant chimeric protein was expressed in bacteria (E. *coli*) and purified according to the procedure described in Example 1.

Example 3: transfection of the chimeric E2GFP-linker-DsRed-monomer protein

The cDNA of the construct obtained in Example 2 was extracted by PCR from the pBR-IBA 2 vector and subcloned into the eukaryotic expression plasmid pcDNA3.1+ (Invitrogen) containing the CMV eukaryotic promoter.

The construct was transfected into two different cell lines:

(i) 3T3 Swiss fibroblast line expressing the human wild-type CFTR (CFTR = cystic fibrosis transmembrane conductance regulator protein) (Galietta, L. J.; Haggie, P. M. & Verkman, A. S. Green fluorescent protein-based halide indicators with improved chloride and iodide affinities. FEBS Lett, 2001, 499, 220-224; Jayaraman, S.; Haggie, P.; Wachter, R. M.; Remington, S. J. & Verkman, A. S. Mechanism and cellular applications of a green fluorescent protein-based halide sensor. J Biol Chem, 2000, 275, 6047-6050);
(ii) HEK293 cell line (human embryonic kidney cells) expressing the $\gamma$-aminobutyric acid A receptor (GABAA) (Wong, G.; Sei, Y. & Skolnick, P. Stable expression of type I gamma-aminobutyric acid A/benzodiazepine receptors in a transfected cell line. Mol Pharmacol, 1992, 42, 996-1003).

The cells indicated in (i) and (ii) were plated onto poly-lysine-coated coverslips (18 mm in diameter) and transfected with 400 ng of plasmid by using the Effectene kit (Quiagen) following the manufacturer's instructions. The cells were grown at 37°C (95% air, 5% $CO_2$) using DMEM and observed 2-3 days after the transfection.

Example 4: Study of the fluorescence properties

The fluorescence properties of the E2GFP and D3GFP mutants were studied, as well as those of the chimeric D3GFP-linker-DsRed-monomer protein of the invention as obtained following the instructions provided in the above examples 1 through 3.

First of all, a study was conducted on how the fluorescence of the two mutants of the Green Fluorescent Protein, E2GFP and D3GFP, changes as a function of the pH. The results thus obtained are depicted in Fig. 2, which shows the excitation spectra of the two mutants E2GFP (A) and D3GFP (B) at different pH values. The data were collected with a Cary Eclipse spectrofluorometer (Varian) under the conditions described below relating to Figure 3, except that the emission wavelength was set at 523 nm and the temperature was adjusted to 20°C. The respective excitation fluorescence ratios between 488 nm and 458 nm and the dependence of such ratios on the pH, modelled according to Grynkiewicz's equation (Grynkiewicz, G., Poenie, M. & Tsien, R. Y., 1985. J. Biol. Chem 260, 3440-3450.) are shown in the panels of figs. 2A and 2B. The pKa of E2GFP and D3GFP are 7.0 $\pm$ 0.15 and 6.3 $\pm$ 0.2, respectively.

Subsequently, a study was conducted on how the fluorescence of the chimeric D3GFP-linker-DsRed-monomer protein changes as a function of the concentration of chlorine ions ([Cl] mM). The results thus obtained are depicted in Fig. 3, which shows the excitation spectrum at different concentrations of chlorine ions. The data were collected with a Cary Eclipse spectrofluorometer (Varian) at a 590 nm fixed emission, at a 5 nm excitation and emission split bandwidth. The temperature was adjusted to 37°C and the ionic strength of the solution was maintained at 1M with $Na_2SO_4$ and NaCl. The ratio between the 514 nm excitation fluorescence and the 543 nm excitation fluorescence (argon and helium/neon laser lines present in the most common confocal microscopes) as a function of $[Cl^-]$ mM, as obtained from the spectra, is shown in the right panel. The curve shown in the right panel was obtained by using Grynkiewicz's equation (Grynkiewicz, G., Poenie, M. & Tsien, R. Y., 1985. J. Biol. Chem 260, 3440-3450.).

Finally, *in vivo* calibrations of the D3GFP-linker-DsRed-monomer sensor were performed according to the pH (fig. 3) and $[Cl^-]$ mM.

The chimeric D3GFP-linker-DsRed-monomer protein was transfected into the 3T3 and HEK293 cell lines as described in Example 3. The intracellular concentration of chlorine ions was maintained at 90 mM.

The measurements were performed with a TCS SP2 confocal laser scanning microscope (Leica Microsystems) by using a 40X/1.25 NA oil immersion objective (Leica HCX PL APO) and a temperature-controlled incubation chamber. The analysis was carried out at three excitation wavelengths, based on the sequential acquirement of images with: (ch02) excitation at 458 nm and emission detection at 500-550 nm, (ch03) excitation at 488 nm and emission detection at 500-550 nm, and (ch05) excitation at 543 nm and emission detection at 570-680 nm.

The pinhole size was maintained at 1 Airy unit, the scanning rate at 400 Hz and the image size at 512 x 512 pixels.

The results obtained at three different pH values are shown in fig. 4 (higher line: pH = 4.95; middle line: pH = 6.64; lower line: pH = 8.32), as they are at the three above-indicated excitation wavelengths (left column: $\lambda$ex 458 nm; middle column: $\lambda$ex 488 nm; right column: $\lambda$ex 543 nm).

From this figure it can be seen that the excitation fluorescence at the 458 nm and 488 nm wavelengths increases when the pH increases, whereas the excitation fluorescence remains constant at the wavelength of 543 nm, therefore this value is used for the normalisation.

The $R_1$ = ch03/ch02 ratio is correlated to the intracellular pH through Grynkiewicz's equation (cf. fig. 5), whereas

the $R_2$ = (ch03 + ch02)/(ch05) ratio is correlated to the intracellular concentration of chlorine ions (cf. fig. 6).

The variation of the $R_1$ = ch03/ch02 parameter as a function of the pH between about 4.5 < pH < 8.5 is shown in fig. 5.

The variation of the $R_2$ = (ch03 + ch02)/(ch05) parameter as a function of $[Cl^-]$ mM at two pH values, 6.3 and 7.35, is shown in fig. 6.

## Claims

1. A fluorescent protein having the amino acid sequence of the *Aequorea victoria* wild-type GFP protein except for the following amino acid substitutions:

   (i) substitution of the phenylalanine residue (F) at position 64 with a leucine residue (L) [F64L];
   (ii) substitution of the serine residue (S) at position 65 with a threonine residue (T) [S65T];
   (iii) substitution of the threonine residue (T) at position 203 with a tyrosine residue (Y) [T203Y];
   (iv) substitution of the valine residue (V) at position 224 with a leucine residue (L) [V224L];
   (v) substitution of the histidine residue (H) at position 231 with a leucine residue (L) [H231L].

2. The fluorescent protein according to claim 1, having the amino acid sequence SEQ ID NO:3.

3. An isolated nucleic acid comprising a nucleotide sequence encoding for a fluorescent protein according to claim 1 or 2.

4. The isolated nucleic acid according to claim 3, comprising the nucleotide sequence SEQ ID NO:4.

5. A chimeric protein comprising a first fluorescent protein, P1, and a second fluorescent protein, P2, wherein P1 is the fluorescent protein according to claim 1 or 2 and P2 is a fluorescent protein, the fluorescence characteristics of which are independent from both the pH and Chlorine ion concentration, wherein P2 is selected so that there is no substantial overlapping of the P1 and P2 emission and excitation fluorescence spectra.

6. The chimeric protein according to claim 5, wherein P1 and P2 are connected through a peptide linker.

7. A chimeric protein according to claim 5 or 6, wherein the P2 protein is selected from the group consisting of DsRFPm, mTangerine, mStrawberry, mCherry, mGrapel, mRaspberry, mPlum, TdTomato, dTomato, DsRed (Tsien Lab), HcRed, Red EosFP.

8. The chimeric protein according to claim 7, wherein P2 is the monomeric DsRed-monomer protein.

9. A chimeric protein according to any of claims 5 to 8, wherein the peptide linker is the amino acid sequence SEQ ID NO:5.

10. An isolated nucleic acid comprising a nucleotide sequence encoding for a chimeric protein according to any of claims 5 to 9.

11. An expression vector comprising an isolated nucleic acid according to any of claims 2, 3 or 10.

12. A transformed host cell comprising an expression vector according to claim 11.

13. The transformed host cell according to claim 12, which is a prokaryotic cell.

14. The transformed host cell according to claim 12, which is an eukaryotic cell.

15. The use of the chimeric protein according to any of claims 5 to 9 as a sensor for the combined measurement of the pH and Chlorine ion concentration in a sample.

16. The use according to claim 15, for the combined measurement of the pH and chlorine ion concentration within an eukaryotic or prokaryotic cell.

17. A method for the combined assaying of the pH and Chlorine ion concentration in a sample, comprising the steps of:

   (a) contacting the sample with the chimeric protein according to any one of claims 5 to 9;
   (b) measuring a first parameter, $R_1$, from the excitation spectrum of the chimeric protein, wherein said first parameter $R_1$ varies according to the pH but does not vary according to the Chlorine ion concentration in the sample;
   (c) correlating the value of the above-mentioned $R_1$ parameter with the pH value of the sample;
   (d) measuring a second parameter, $R_2$, from the excitation spectrum of the chimeric protein, wherein said

parameter $R_2$ varies according to both the pH and Chlorine ion concentration;
(e) correlating the value of the above-mentioned $R_2$ parameter with the Chlorine ion concentration in the sample.

**18.** The method according to claim 17, wherein in step (c) the value of the $R_1$ parameter is correlated with the pH value of the sample by comparison to the value of the $R_1$ parameter from one or more reference samples of known pH.

**19.** A method according to any of claims 17 or 18, wherein in step (e) the value of the $R_2$ parameter is correlated with the Chlorine ion concentration value in the sample by comparison to the value of the $R_2$ parameter from one or more reference samples of known Chlorine ion concentration and having approximately the pH value determined in step (c).

**20.** A method according to any of claims 17 to 18, wherein the $R_1$ parameter is represented by the formula:

$$R_1 = \frac{A}{B}$$

wherein A is the fluorescence emitted as a result of a 488 nm wavelength excitation and B is the fluorescence emitted as a result of a 458 nm wavelength excitation.

**21.** A method according to any of claims 17 to 20, wherein the $R_2$ parameter is represented by the formula:

$$R_2 = \frac{(A + B)}{C}$$

wherein A and B are as defined in claim 22 and C is the fluorescence emitted as a result of a 543 nm wavelength excitation.

**22.** The method according to any of claims 17 to 21, wherein in step (a) the chimeric protein is transfected into a cell and wherein the pH and chlorine ion concentration values being measured are the intracellular ones.

**Patentansprüche**

**1.** Fluoreszierendes Protein mit der Aminosäuresequenz des *Aequorea victoria*-Wildtyp-GFP-Proteins mit Ausnahme der folgenden Aminosäuresubstitutionen:

(i) Substitution des Phenylalaninrestes (F) an Position 64 durch einen Leucinrest (L) [F64L];
(ii) Substitution des Serinrestes (S) an Position 65 durch einen Threoninrest (T) [S65T];
(iii) Substitution des Threoninrestes (T) an Position 203 durch einen Tyrosinrest (Y) [T203Y];
(iv) Substitution des Valinrestes (V) an Position 224 durch einen Leucinrest (L) [V224L];
(v) Substitution des Histidinrestes (H) an Position 231 durch einen Leucinrest (L) [H231 L].

**2.** Fluoreszierendes Protein nach Anspruch 1 mit der Aminosäresequenz SEQ ID NO:3.

**3.** Isolierte Nukleinsäure, umfassend eine Nukleotidsequenz, die für ein fluoreszierendes Protein nach Anspruch 1 oder 2 codiert.

**4.** Isolierte Nukleinsäure nach Anspruch 3, umfassend die Nukleotidsequenz SEQ ID NO:4.

**5.** Chimäres Protein, umfassend ein erstes fluoreszierendes Protein, P1, und ein zweites fluoreszierendes Protein, P2, worin P1 das fluoreszierende Protein nach Anspruch 1 oder 2 ist und P2 ein fluoreszierendes Protein ist, dessen Fluoreszenzcharakteristika unabhängig von sowohl dem pH-Wert als auch der Chlorionen-Konzentration sind, worin P2 so ausgewählt ist, dass es keine wesentliche Überlappung der P1- und P2-Emissions- und Anregungsfluoreszenzspektren gibt.

**6.** Chimäres Protein nach Anspruch 5, worin P1 und P2 durch einen Peptid-Linker verbunden sind.

**7.** Chimäres Protein nach Anspruch 5 oder 6, worin das P2-Protein ausgewählt ist aus der Gruppe, bestehend aus DsRFPm, mTangerine, mStrawberry, mCherry, mGrape1, mRasperry, mPlum, TdTomato, dTomato, DsRed (Tsien Lab), HcRed, Red EosFP.

**8.** Chimäres Protein nach Anspruch 7, worin P2 das monomere DsRed-Proteinmonomer ist.

**9.** Chimäres Protein nach einem der Ansprüche 5 bis 8, worin der Peptidlinker die Aminosäuresequenz SEQ ID NO:5 ist.

**10.** Isolierte Nukleinsäure, umfassend eine Nukleotidsequenz, die für ein chimäres Protein nach einem der Ansprüche 5 bis 9 codiert.

**11.** Expressionsvektor, umfassend eine isolierte Nukleinsäure nach einem der Ansprüche 2, 3 oder 10.

**12.** Transformierte Wirtszelle, umfassend einen Expressionsvektor nach Anspruch 11.

**13.** Transformierte Wirtszelle nach Anspruch 12, die eine prokariontische Zelle ist.

**14.** Transformierte Wirtszelle nach Anspruch 12, die eine eukariontische Zelle ist.

**15.** Verwendung des chimären Proteins nach einem der Ansprüche 5 bis 9 als ein Sensor für die kombinierte Messung des pH-Werts und der Chlorionen-Konzentration in einer Probe.

**16.** Verwendung nach Anspruch 15 zur kombinierten Messung des pH-Werts und der Chlorionen-Konzentration in einer eukariontischen oder prokariontischen Zelle.

**17.** Verfahren zum kombinierten bestimmen des pH-Werts und der Chlorionen-Konzentration in einer Probe, umfassend die Schritte:

(a) In-Kontakt-bringen der Probe mit dem chimären Protein nach einem der Ansprüche 5 bis 9;
(b) Messen eines ersten Parameters, $R_1$, aus dem Anregungsspektrum des chimären Proteins, worin der erste Parameter $R_1$ entsprechend des pH-Werts jedoch nicht entsprechend der Chlorionen-Konzentration in der Probe variiert;
(c) Korrelieren des Werts des oben genannten $R_1$-Parameters mit dem pH-Wert der Probe;
(d) Messen eines zweiten Parameters, $R_2$, aus dem Anregungsspektrum des chimären Proteins, worin der zweite Parameter, $R_2$, sowohl entsprechend pH-Wert als auch Chlorionen-Konzentration variiert;
(e) Korrelieren des Werts des oben genannten $R_2$-Parameters mit der Chlorionen-Konzentration in der Probe.

**18.** Verfahren nach Anspruch 17, worin in Schritt (c) der Wert des $R_1$-Parameters mit dem pH-Wert der Probe durch Vergleich des Werts des $R_1$-Parameters von einer oder mehreren Referenzproben mit bekanntem pH-Wert korreliert wird.

**19.** Verfahren nach einem der Ansprüche 17 oder 18, worin in Schritt (e) der Wert des $R_2$-Parameters korreliert wird mit dem Chlorionen-Konzentrationswert in der Probe durch Vergleich des Werts des $R_2$-Parameters von einer oder mehreren Referenzproben mit bekannter Chlorionen-Konzentration und mit ungefähr dem pH-Wert, der in Schritt (c) bestimmt wurde.

**20.** Verfahren nach einem der Ansprüche 17 bis 18, worin der $R_1$-Parameter durch die Formel:

$$R_1 = \frac{A}{B}$$

dargestellt wird, worin A die Fluoreszenz ist, die als ein Ergebnis einer Anregung mit einer Wellenlänge von 488 nm emittiert wird, und B die Fluoreszenz ist, die als ein Ergebnis einer Anregung mit einer Wellenlänge von 458 nm emittiert wird.

**21.** Verfahren nach einem der Ansprüche 17 bis 20, worin der $R_2$-Parameter durch die Formel:

$$R_2 = \frac{(A+B)}{C}$$

dargestellt wird, worin A und B wie in Anspruch 22 definiert sind und C die Fluoreszenz ist, die als ein Ergebnis einer Anregung mit einer Wellenlänge von 543 nm emittiert wird.

**22.** Verfahren nach einem der Ansprüche 17 bis 21, worin in Schritt (a) das chimere Protein in eine Wirtszelle transfiziert wird und worin der gemessene pH-Wert und die gemessenen Chlorionen-Konzentrationswerte intrazelluläre sind.

## Revendications

**1.** Protéine fluorescente présentant la séquence d'acides aminés de la GFP de type sauvage d'*Aequorea victoria,* exception faite des substitutions d'acides aminés suivantes :

(i) substitution du résidu phénylalanine (F) en position 64 par un résidu leucine (L) [F64L] ;
(ii) substitution du résidu serine (S) en position 65 par un résidu thréonine (T) [S65T] ;
(iii) substitution du résidu thréonine (T) en position 203 par un résidu tyrosine (Y) [T203Y] ;
(iv) substitution du résidu valine (V) en position 224 par un résidu leucine (L) [V224L] ;
(v) substitution du résidu histidine (H) en position 231 par un résidu leucine (L) [H231L] ;

**2.** Protéine fluorescente conforme à la Revendication 1, présentant la séquence d'acides aminés SEQ ID NO:3.

**3.** Acide nucléique purifié comprenant une séquence de nucléotides codant une protéine fluorescente conforme à la Revendication 1 ou 2.

**4.** Acide nucléique purifié conforme à la Revendication 3, comprenant la séquence de nucléotides SEQ ID NO:4.

**5.** Protéine chimère comprenant une première protéine fluorescente $P_1$, et une seconde protéine fluorescente $P_2$, $P_1$ étant la protéine fluorescente conforme à la Revendication 1 ou 2 et $P_2$ étant une protéine fluorescente dont les caractéristiques de fluorescence sont indépendantes du pH comme de la concentration en ions chlores, $P_2$ étant choisie de sorte qu'il n'y ait pas de chevauchement substantiel entre les spectres d'émission et d'excitation de fluorescence de $P_1$ et $P_2$.

**6.** Protéine chimère conforme à la Revendication 5, $P_1$ et $P_2$ étant reliées par un adaptateur peptidique.

**7.** Protéine chimère conforme à la Revendication 5 ou 6, la protéine $P_2$ étant choisie dans le groupe comprenant DsRFPm, mTangerine, mStrawberry, mCherry, mGrapel, mRaspberry, mPlum, TdTomato, dTomato, DsRed (Tsien Lab), HcRed, Red EosFP.

**8.** Protéine chimère conforme à la Revendication 7, $P_2$ étant la protéine monomère DsRed-monomère.

**9.** Protéine chimère conforme à l'une quelconque des Revendications 5 à 8, l'adaptateur peptidique étant de séquence d'acides aminés SEQ ID NO:5.

**10.** Acide nucléique purifié comprenant une séquence de nucléotides codant une protéine chimère conforme à l'une quelconque des Revendications 5 à 9.

**11.** Vecteur d'expression comprenant un acide nucléique purifié conforme à l'une quelconque des Revendications 2, 3 ou 10.

**12.** Cellule hôte transformée comprenant un vecteur d'expression conforme à la Revendication 11.

**13.** Cellule hôte transformée conforme à la Revendication 12, qui est une cellule procaryote.

**14.** Cellule hôte transformée conforme à la Revendication 12, qui est une cellule eucaryote.

**15.** Utilisation de la protéine chimère conforme à l'une quelconque des Revendications 5 à 9 comme capteur pour la mesure conjointement du pH et de la concentration en ions chlores dans un échantillon.

**16.** Utilisation selon la Revendication 15 pour la mesure conjointement du pH et de la concentration en ions chlores à l'intérieur d'une cellule eucaryote ou procaryote.

**17.** Procédé de dosage conjointement du pH et de la concentration en ions chlores dans un échantillon, comprenant les étapes consistant à:

(a) mettre en contact l'échantillon avec la protéine chimère conforme à l'une quelconque des Revendications 5 à 9;
(b) mesurer un premier paramètre $R_1$ d'après le spectre d'excitation de la protéine chimère, ledit premier paramètre $R_1$ variant selon le pH, mais ne variant pas selon la concentration en ions chlores dans l'échantillon;
(c) établir une corrélation entre la valeur du paramètre $R_1$ mentionné ci-dessus et la valeur du pH de l'échantillon;
(d) mesurer un second paramètre $R_2$ d'après le spectre d'excitation de la protéine chimère, ledit second paramètre $R_2$ variant selon le pH comme selon la concentration en ions chlores;
(e) établir une corrélation entre la valeur du paramètre $R_2$ mentionné ci-dessus et la concentration en ions chlores dans l'échantillon.

**18.** Procédé selon la Revendication 17, la valeur du paramètre $R_1$ dans l'étape (c) étant corrélée à la valeur de pH de l'échantillon par comparaison à la valeur du paramètre $R_1$ provenant d'un ou de plusieurs échantillons de référence, de pH connu.

**19.** Procédé selon l'une quelconque des Revendications 17 ou 18, la valeur du paramètre $R_2$ dans l'étape (e) étant corrélée à la valeur de concentration en ions chlores dans l'échantillon par comparaison à la valeur du paramètre $R_2$ provenant d'un ou de plusieurs échantillons de référence de concentration en ions chlores connue et présentant approximativement une valeur de pH équivalente à celle déterminée dans l'étape (c).

**20.** Procédé selon l'une quelconque des Revendications 17 à 18, le paramètre $R_1$ étant représenté par la formule :

$$R_1=A/B$$

A représentant la fluorescence émise suite à une excitation à une longueur d'onde de 488 nm et B représentant la fluorescence émise suite à une excitation à une longueur d'onde de 458 nm.

**21.** Procédé selon l'une quelconque des Revendications 17 à 20, le paramètre $R_2$ étant représenté par la formule :

$$R_2=(A+B)/C$$

A et B étant tels que définis en Revendication 22 et C représentant la fluorescence émise suite à une excitation à une longueur d'onde de 543 nm.

**22.** Procédé selon l'une quelconque des Revendications 17 à 21, la protéine chimère étant, dans l'étape (a), transfectée dans une cellule et les valeurs de pH et de concentration en ions chlores qui sont mesurées étant les valeurs intracellulaires.

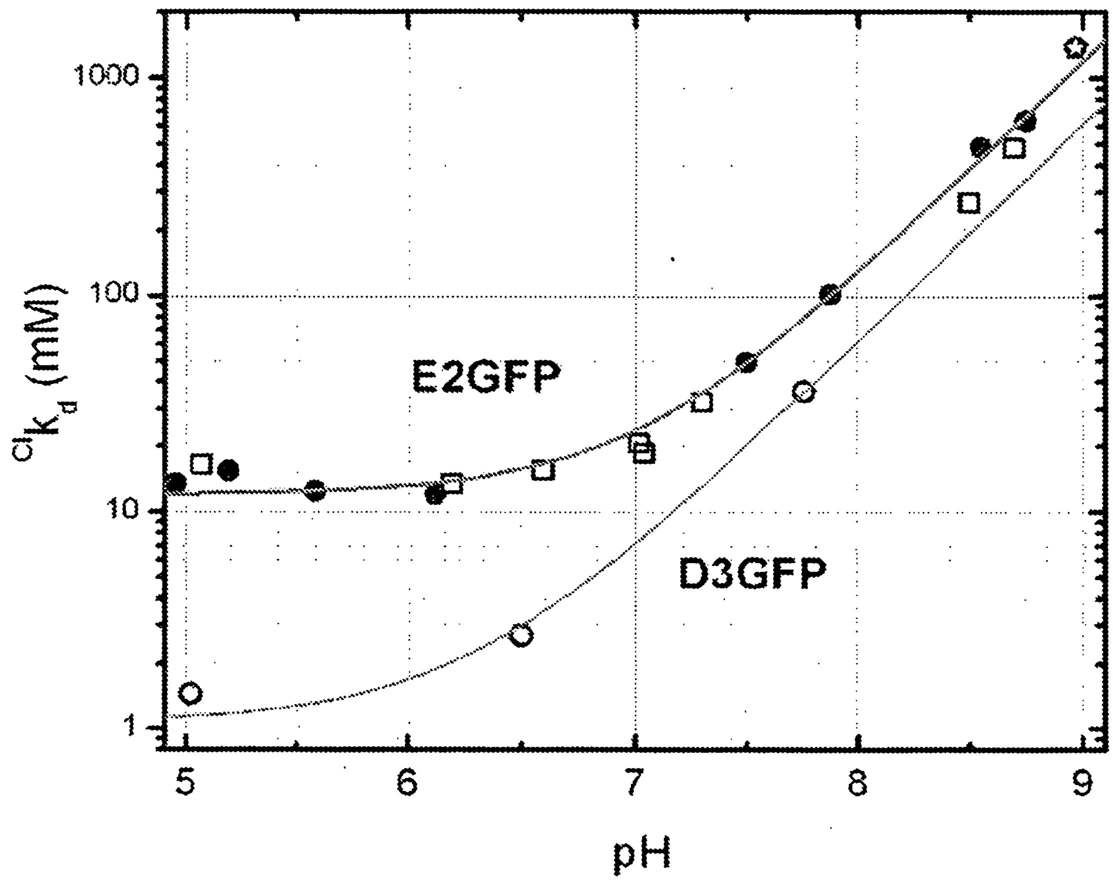


FIG.1

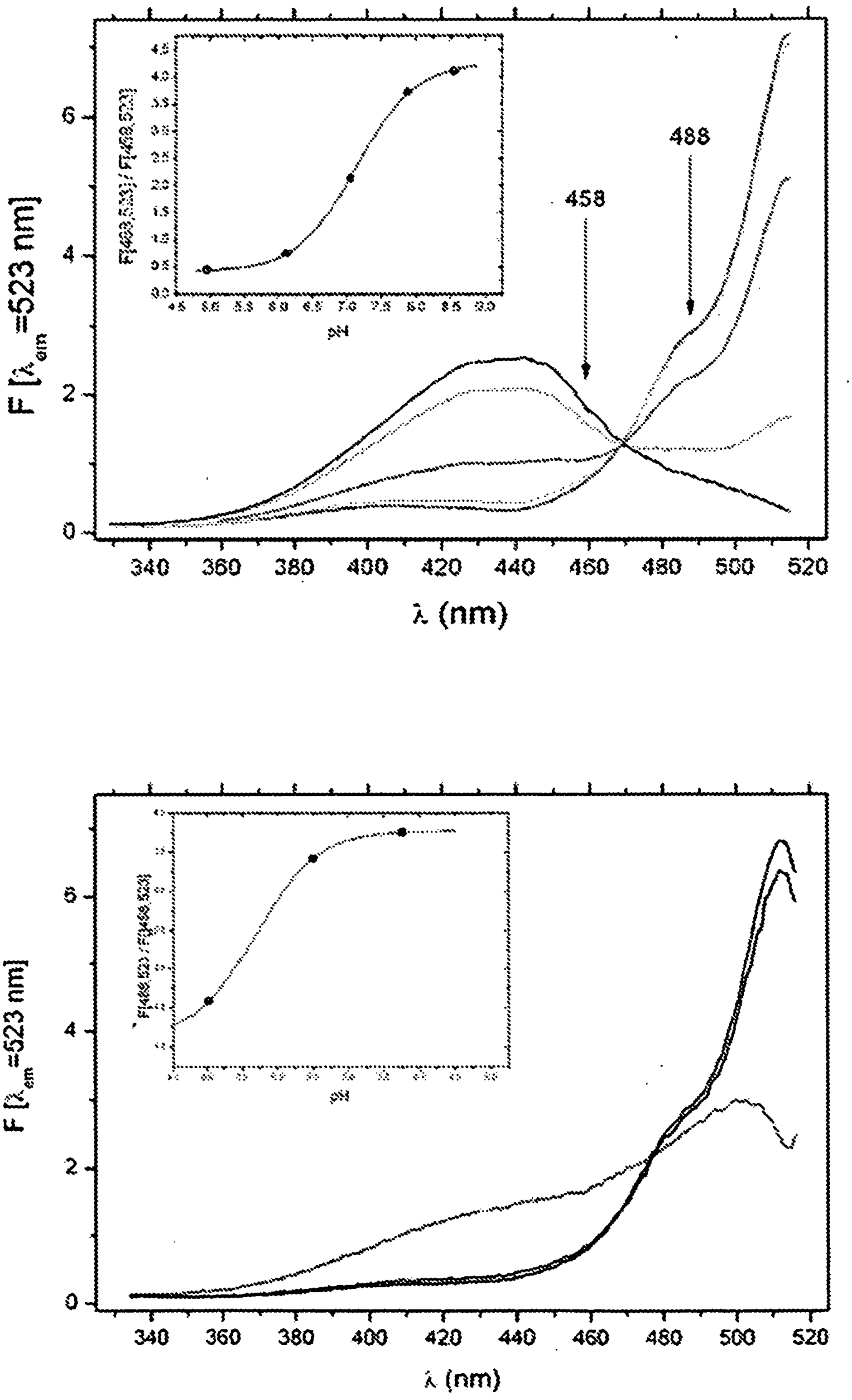
F [λem=523 nm]
6
4
2
0
458
488
340 360 380 400 420 440 460 480 500 520
λ (nm)
F [λem=523 nm]
6
4
2
0
340 360 380 400 420 440 460 480 500 520
λ (nm)

FIG.2

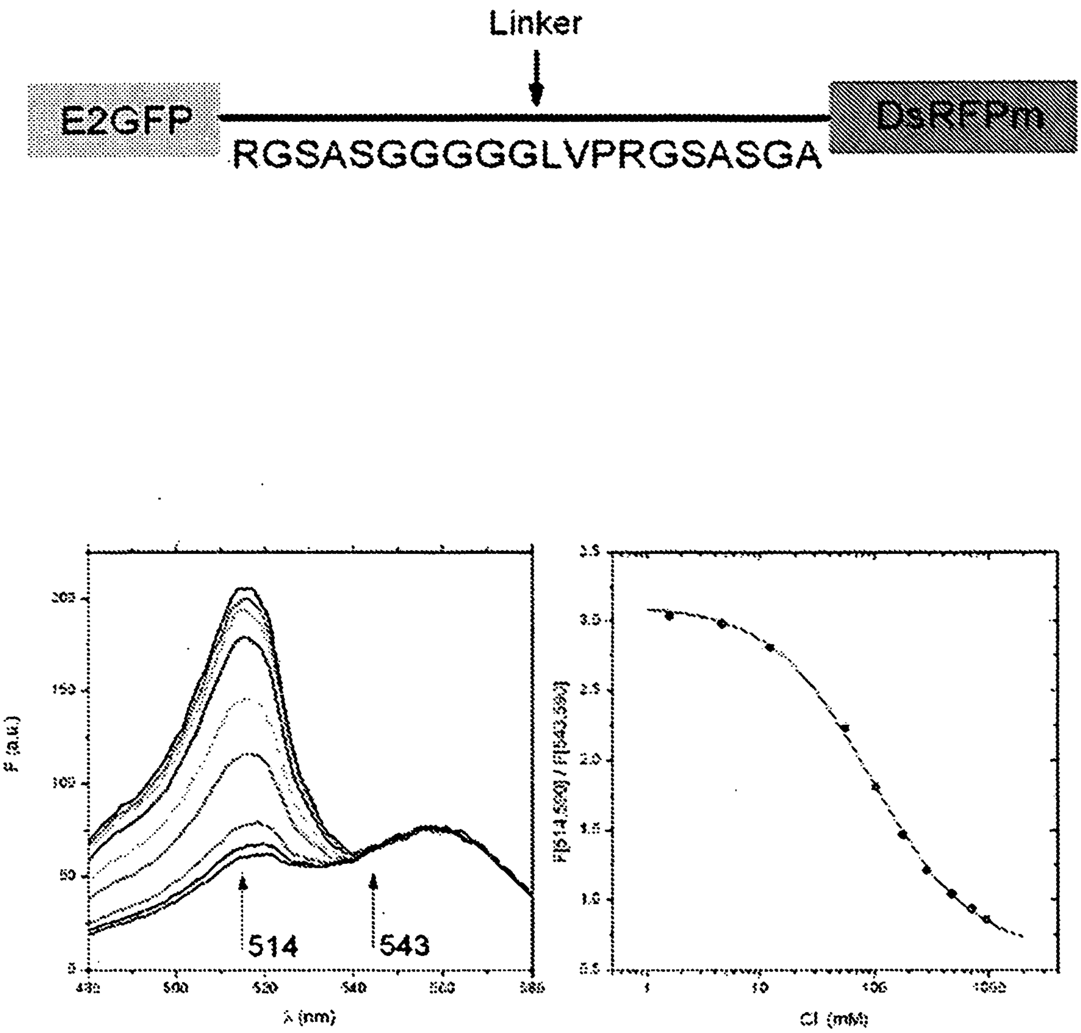


FIG.3

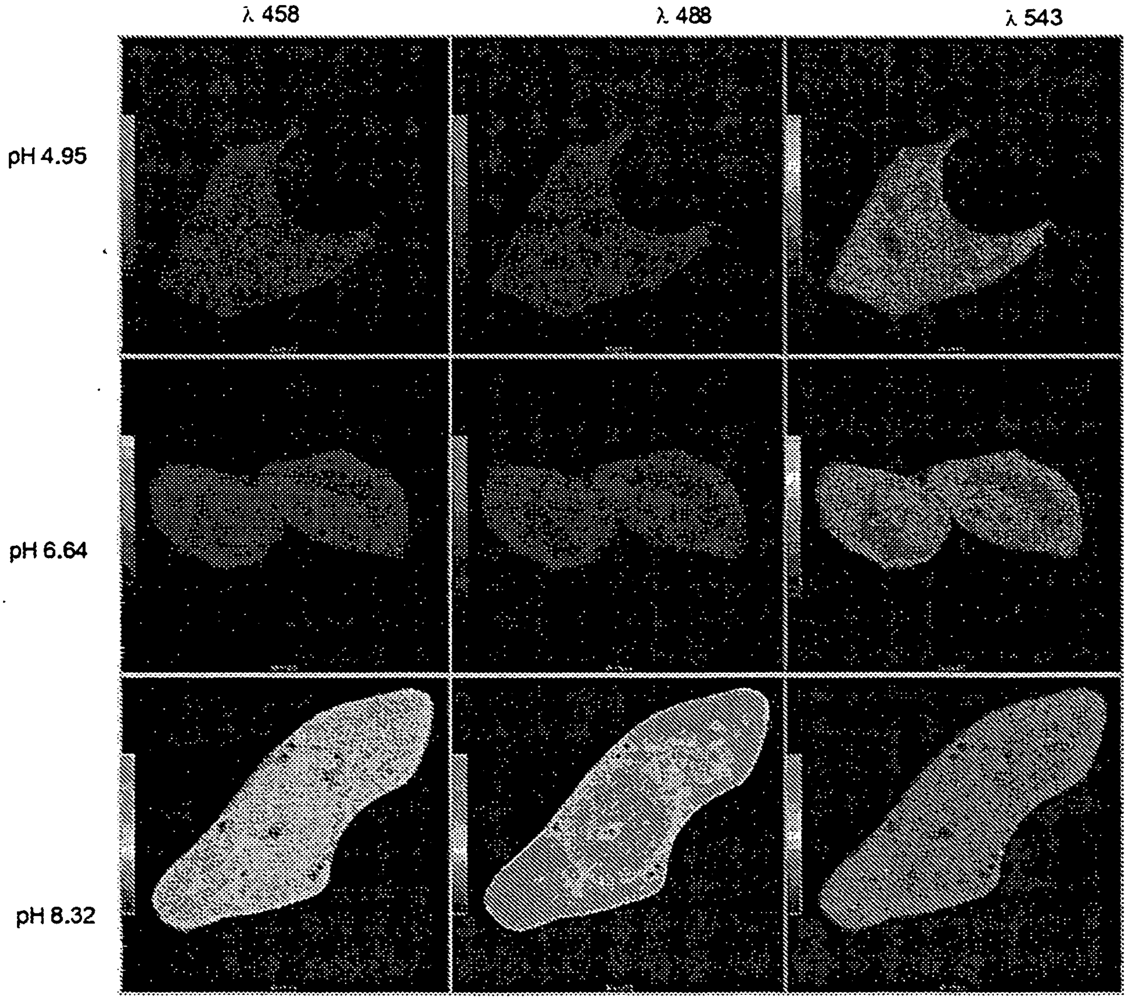


FIG.4

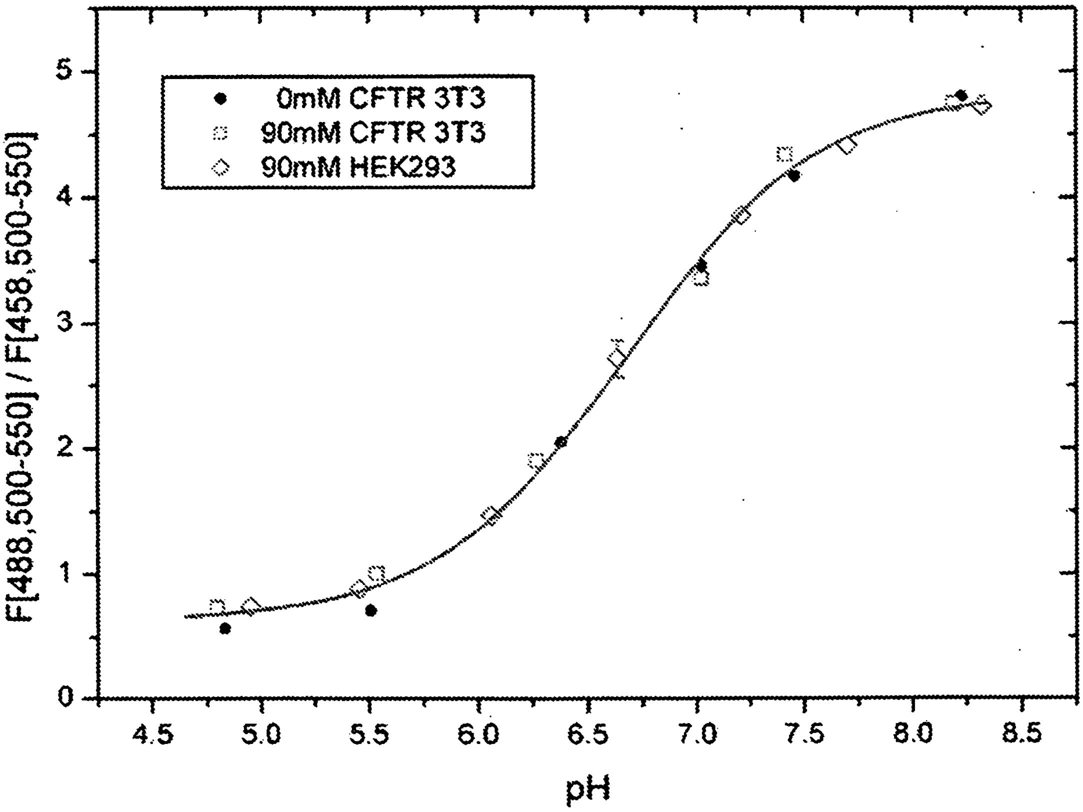
0mM CFTR 3T3
90mM CFTR 3T3
90mM HEK293
F[488,500-550] / F[458,500-550]
0
1
2
3
4
5
4.5
5.0
5.5
6.0
6.5
7.0
7.5
8.0
8.5
pH

FIG.5

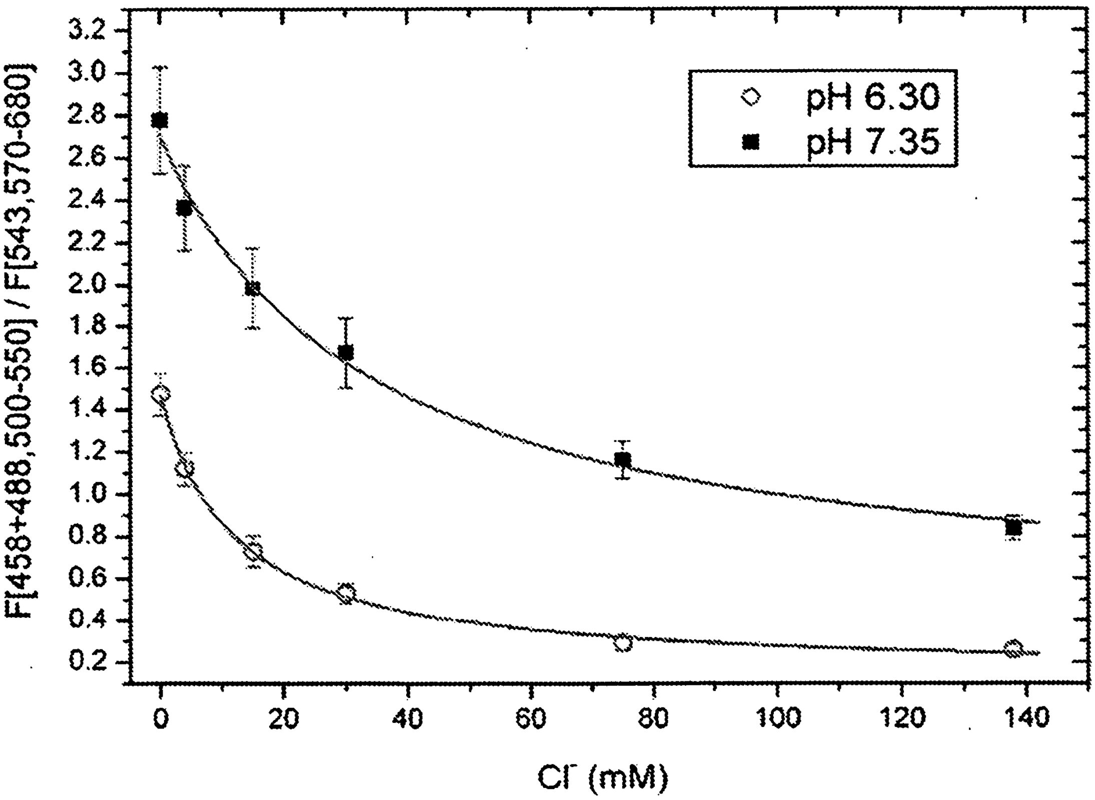
3.2
3.0
2.8
2.6
2.4
2.2
2.0
1.8
1.6
1.4
1.2
1.0
0.8
0.6
0.4
0.2
F[458+488,500-550] / F[543,570-680]
pH 6.30
pH 7.35
0
20
40
60
80
100
120
140
Cl⁻ (mM)

FIG.6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- IT 1320791
- EP 1514922 A


### Non-patent literature cited in the description


- **Arosio D ; Garau G ; Ricci F ; Marchetti L ; Bizzarri R ; Nifosi R ; Beltram F.** *Biophys J.,* 13 April 2007, vol. 93 (1), 232-44
- **Shaner, N. C. ; Campbell, R. E. ; Steinbach, P. A. ; Giepmans, B. N. G. ; Palmer, A. E. ; Tsien, R. Y.** Improved monomeric red, orange and yellow fluorescent proteins derived from Discosoma sp. red fluorescent protein. *Nat Biotechnol,* 2004, vol. 22, 1567-1572
- **Gurskaya, N. G. ; Fradkov, A. F. ; Terskikh, A. ; Matz, M. V. ; Labas, Y. A. ; Martynov, V. I. ; Yanushevich, Y. G. ; Lukyanov, K. A. ; Lukyanov, S. A.** GFP-like chromoproteins as a source of far-red fluorescent proteins. *FEBS Lett,* 2001, vol. 507, 16-20
- **Wiedenmann, J. ; Ivanchenko, S. ; Oswald, F. ; Schmitt, F. ; Röcker, C. ; Salih, A. ; Spindler, K. ; Nienhaus, G. U.** EosFP, a fluorescent marker protein with UV-inducible green-to-red fluorescence conversion. *Proc Natl Acad Sci U S A,* 2004, vol. 101, 15905-15910
- **Galietta, L. J. ; Haggie, P. M. ; Verkman, A. S.** Green fluorescent protein-based halide indicators with improved chloride and iodide affinities. *FEBS Lett,* 2001, vol. 499, 220-224
- **Jayaraman, S. ; Haggie, P. ; Wachter, R. M. ; Remington, S. J. ; Verkman, A. S.** Mechanism and cellular applications of a green fluorescent protein-based halide sensor. *J Biol Chem,* 2000, vol. 275, 6047-6050
- **Wong, G. ; Sei, Y. ; Skolnick, P.** Stable expression of type I gamma-aminobutyric acid A/benzodiazepine receptors in a transfected cell line. *Mol Pharmacol,* 1992, vol. 42, 996-1003
- **Grynkiewicz, G. ; Poenie, M ; Tsien, R. Y.** *J. Biol. Chem,* 1985, vol. 260, 3440-3450
- **Grynkiewicz, G. ; Poenie, M. ; Tsien, R. Y.** *J. Biol. Chem,* 1985, vol. 260, 3440-3450